(19) Europäisches Patentamt
European Patent Office
Office européen des brevets

(11) Publication number: **0 049 144**
**B1**

(12) **EUROPEAN PATENT SPECIFICATION**

(45) Date of publication of patent specification: **29.08.84**

(51) Int. Cl.$^3$: **C 07 D 239/54,** A 61 K 31/505

(21) Application number: **81304464.1**

(22) Date of filing: **28.09.81**

(54) **5-Fluoro uracil derivatives.**

(30) Priority: **01.10.80 GB 8031721**
**25.07.81 GB 8123032**

(43) Date of publication of application:
**07.04.82 Bulletin 82/14**

(45) Publication of the grant of the patent:
**29.08.84 Bulletin 84/35**

(84) Designated Contracting States:
**CH DE FR IT LI NL**

(56) References cited:
**GB-A-1 393 863**
**GB-A-1 542 053**
**US-A-4 124 765**

(73) Proprietor: **BEECHAM GROUP PLC**
**Beecham House Great West Road**
**Brentford Middlesex (GB)**

(72) Inventor: **Harnden, Michael Raymond**
**47 Guildford Road**
**Horsham Sussex (GB)**
Inventor: **Bailey, Stuart**
**14 Overdale**
**Dorking Surrey (GB)**
Inventor: **Shanks, Colin Thomas**
**12 Meath Green Avenue**
**Horley Surrey (GB)**

(74) Representative: **Russell, Brian John et al**
**European Patent Attorney Beecham Pharmaceuticals Great Burgh Yew Tree Bottom Road**
**Epsom Surrey, KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 049 144 B1

**Description**

This invention relates to certain new substituted uracils which have anti-tumour activity.

It is known to react trimethylsilylated uracil derivatives with acetals in the presence of Lewis acids, such as $SnCl_4$, to give 1-(1-methoxyalkyl) uracils [see for example U. Niedballa and H. Vorbruggen *J. Org. Chem., 41,* 2084 (1976) and *39* (1974); Japanese Patent Nos. 1,113,882; 3,023,986; 3,023,987; 2,059,171; 2,059,172; 2,083,386; 2,128,384; 4,038,344].

U.S. Patent No. 4,124,765 discloses 5-fluorouracil derivatives of the general formula (A):

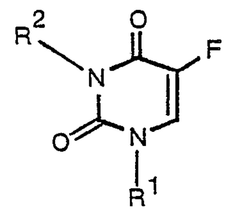

(A)

wherein each of $R^1$ and $R^2$ is hydrogen or

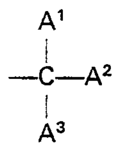

wherein $A_1$, $A_2$ and $A_3$ are independently selected from hydrogen; alkoxy ($R_3O$—, where $R_3$ is straight or branched chain alkyl of from 1 to 10 carbon atoms or cycloalkyl of from 4 to 6 carbon atoms) and phenyloxy ($R_4O$—, wherein $R_4$ is a phenyl group); the alkoxy and phenyloxy being unsubstituted or substituted with fluoro, chloro, bromo, iodo, hydroxy, phenyloxy, acetoxy, alkoxy of 1 to 5 carbon atoms, or alkyl or alkenyl of 1 to 5 carbon atoms and which may be substituted with fluoro, chloro, bromo, iodo, methoxy or hydroxy; at least one of $A_1$ and $A_2$ and $A_3$ being other than hydrogen;
and with the proviso that $R^1$ and $R^2$ are not simultaneously hydrogen. These derivatives are said to have anti-tumour activity.

U.K. Patent Specification No. 1,542,053 discloses further 5-fluorouracil derivatives of the general formula (B);

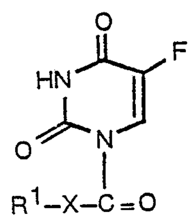

(B)

wherein X is oxygen or sulphur and $R^1$ is a straight or branched-chain alkyl group having 1 to 15 carbon atoms, a cycloalkyl group having 4 to 8 carbon atoms, a straight or branched-chain alkenyl group having 2 to 5 carbon atoms, a straight or branched-chain alkynyl group having 2 to 15 carbon atoms or an aryl group in which the alkyl, cycloalkyl, alkenyl, alkynyl or aryl group is unsubstituted or substituted with one or more of a halogen atom (i.e. F, Cl, Br or I), a hydroxy group, a straight or branched-chain alkenyl or alkynyl group having 2 to 6 carbon atoms, a straight or branched-chain alkoxy group having the formula $R^2$—O— or an acyloxy group having the formula $R^2COO$—, wherein $R^2$ represents a straight or branched-chain alkyl group having 1 to 6 carbon atoms, or an aryl or aryloxy group which is unsubstituted or substituted with one or more of a halogen atom, an alkyl group having 1 to 5 carbon atoms or an alkoxy group having 1 to 5 carbon atoms. These derivatives are also said to have anti-tumour activity.

We have now discovered a class of novel 5-fluoro substituted uracils which possess anti-tumour activity at doses well below those at which they are toxic for mammals. These novel substituted uracils are also useful as intermediates in the preparation of other anti-tumour agents.

According to the present invention there is provided a compound of formula (I):

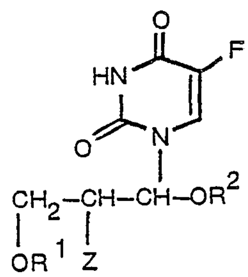

(I)

in which $R^1$ is hydrogen or $C_{1-6}$ alkanoyl, $R^2$ is $C_{1-6}$ alkyl, and Z is hydrogen or $OR^1$.

Preferably, $R^2$ is methyl and $R^1$ is acetyl or hydrogen.

Suitably, when Z is hydrogen or hydroxyl, $R^1$ is hydrogen.

The compounds of formula (I) in which $R^1$ is $C_{1-6}$ alkanoyl may be prepared by reacting a trialkylsilylated uracil derivative of formula (II):

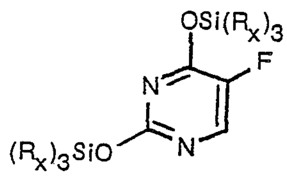

(II)

in which $R_x$ is a $C_{1-4}$ alkyl group, preferably methyl, with a compound of formula (III):

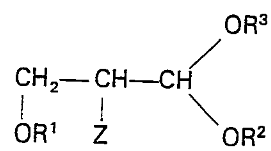

(III)

in which $R^1$ is $C_{1-6}$ alkanoyl, Z and $R^2$ are as defined in formula (I), and $R^3$ is $C_{1-6}$ alkyl or $C_{1-6}$ alkanoyl, in the presence of a Lewis acid, preferably $SnCl_4$.

The reaction is suitably carried out in the presence of anhydrous acetonitrile under an atmosphere of dry nitrogen.

Preferably, $R^3$ is $C_{1-6}$ alkanoyl, suitably acetyl. A preferred group for $R^2$ is methyl. Compounds of formula (III) wherein $R^3$ is $C_{1-6}$ alkanoyl are novel intermediates and form a further aspect of the present invention.

Compounds of formula (I) in which $R^1$ is hydrogen and Z is hydroxyl may be prepared by hydrolysing the compounds of formula (I) in which $R^1$ is $C_{1-6}$ alkanoyl and Z is $OR^1$, preferably using a mixture of methanol and ammonia. In a similar manner, compounds of formula (I) in which $R^1$ and Z are both hydrogen may be prepared by hydrolysing compounds of formula (I) in which $R^1$ is $C_{1-6}$ alkanoyl and Z is hydrogen.

The crude reaction products may be purified by short-path column chromatography.

The compounds of formula (II) may themselves be prepared by treating 5-fluoro uracil of formula (IV):

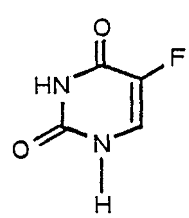

(IV)

with hexamethyldisilazane and/or a trialkylhalo-silane, preferably trimethylchlorosilane. The reaction is suitably carried out in an organic solvent, preferably pyridine.

Compounds of formula (III) in which $R^1$ and $R^3$ are each $C_{1-6}$ alkanoyl may be prepared by:

(a) treating a compound of formula (V):

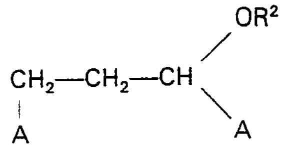

(V)

wherein A is a halogen atom, preferably chlorine, and $R^2$ is as defined in formula (I), with a salt of a $C_{1-6}$ alkanoic acid, or

(b) treating a compound of formula (VI)

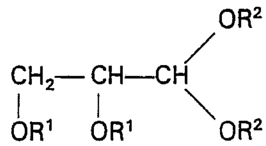

(VI)

wherein $R^2$ is as defined in formula (I), with a $C_{1-6}$ alkanoic acid anhydride.

Process (a) is suitably carried out in the presence of dry acetonitrile and a solubilising agent, such as 18-crown-6, for the alkanoic acid salt.

Process (b) is suitably carried out in the absence of a solvent or diluent, preferably in the presence of a catalytic amount of mineral acid such as sulphuric acid.

The compounds of formula (I) have two asymmetric carbon atoms, marked with asterisks in the following formula:

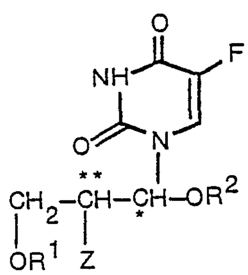

(I)

The compounds therefore exist in four stereoisomeric forms, and the present invention encompasses all stereoisomers alone or admixed with other stereoisomers in any proportion.

In a further aspect of the invention, there is provided a pharmaceutical composition which comprises a compound of the formula (I) together with a pharmaceutically acceptable carrier or excipient.

Compositions which may be given by the oral route may be compounded in the form of syrups, tablets and capsules. When the composition is in the form of a tablet, any pharmaceutical carrier suitable for formulating such solid compositions may be used, for example magnesium stearate, starch, lactose, glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added to form syrups.

The compounds may also be presented with a sterile liquid carrier for injection.

Preferably the compositions of this invention are in unit dosage form or in some other form that the patient may administer to himself a single dose. A suitable dosage unit might contain from 50 mg to 1 g of active ingredient. For example 100 to 500 mg. Such doses may be administered 1 to 4 times a day or more usually 2 or 3 times a day. The effective dose of compound depends on the particular compound employed, but is in general in the range of from 1.0 mg/kg/day to 20 mg/kg of body weight per day or more usually 2.0 mg/kg/day to 10 mg/kg/day.

The following examples illustrate the invention:

Example 1

*1-(2,3-Diacetoxy-1-methoxypropyl)-5-fluorouracil*

Method A

The preparation of 2,4-bis-O-trimethylsilyl-5-fluorouracil was accomplished by treatment of 5-fluorouracil (2.40 g, 18.5 mMol) with hexamethyldisilazane (50 mL) at 160°C for 20 h. Removal of the excess silylating agent under reduced pressure afforded a mobile oil, which was used directly in the subsequent reaction.

The bis-trimethylsilyl derivative (5.28 g, 18.5 mmol) was dissolved in anhydrous acetonitrile (150 mL) and to this solution was added 2,3-diacetoxy-1,1-dimethoxypropane (4.50 g, ≈ 20 mmol). The resultant solution was cooled to −78°C under nitrogen and anhydrous stannic chloride (1 mL) added directly by syringe. The mixture was stirred at −78°C for 15 min and then slowly allowed to attain room temperature. The reaction was subsequently stirred at 25°C for 20 h. under nitrogen.

The volume of the reaction mixture was reduced to approximately 30 mL and to this was added cautiously dilute sodium hydrogen carbonate solution (150 mL). The aqueous mixture was extracted with chloroform (2 x 100 mL) and the organic phase dried ($MgSO_4$) and evaporated under reduced pressure, affording the crude reaction products.

The titled product (3.66 g, 60%), isolated as a mixture of diastereoisomers by short-path column chromatography, using 10—40% acetone in *n*-hexane as eluent, had m.p. 132—134°C; $\nu_{max}$(KBr) 1755, 1725, 1695, 1240, 1215 $cm^{-1}$; $^1$Hn.m.r. [$CDCl_3$] $\delta$ 2.00 (6H, d, 2x $CH_3CO_2$), 3.28 (3H, d, $CH_3O$), 3.95—4.40 (2H, m, $CH_2$), 5.15—5.40 (1H, m, CH) 5.55—5.75 (1H, m, CH), 7.80—8.00 (1H, dd, $C_6H$), 11.90 (1H, br.s, Nh, $D_2O$-exchangeable); m/e (ammonia C.I.) 336 ($MNH_4^+$, 39%). *Anal.* Calcd. for $C_{12}H_{15}N_2O_7$ F:C, 45.28; H, 4.71; N 8.80; F, 5.97. Found: C, 45.39; H, 4.53; N, 8.47; F 5.59%

Method B

2,4-Bis-O-trimethylsilyl-5-fluorouracil was prepared as described in Method A.

To a solution of the bis-trimethylsilyl derivative (5.45 g, 20 mmol) and 1-methoxy-1,2,3-triacetoxypropane (5.00 g, 20 mmol) in anhydrous acetonitrile (100 mL) at −78°C was added stannic chloride (1 mL). The resultant mixture was kept at −78°C for 15 mins and subsequently stirred at 25°C for 18 h under nitrogen.

The reaction volume was reduced to approximately 20 mL and dilute sodium hydrogen carbonate solution (75 mL) added slowly. The aqueous mixture was extracted with chloroform (2x150 mL); the organic extracts combined, dried ($MgSO_4$) and evaporated under reduced pressure.

Short-path column chromatography on silica gel, using 10—40% acetone in *n*-hexane as eluent gave pure 1-(2,3-diacetoxy-1-methoxypropyl)-5-fluorouracil (3.61 g, 57%), with identical physical and spectral properties to the sample prepared by Method A.

Example 2

*1-(2,3-Dihydroxy-1-methoxypropyl)-5-fluorouracil*

To a solution of 1-(2,3-Diacetoxy-1-methoxypropyl)-5-fluorouracil (1.60 g, 5 mmol) in methanol (15 ml) was added 0.88 ammonia solution (60 mL) and the resultant mixture stirred overnight at 25°C. The methanol and water were removed under reduced pressure, affording the crude reaction products.

Short-path column chromatography, using 25-50% acetone in *n*-hexane as eluent, and subsequent crystallisation from methanol-acetone-*n*-hexane (1:2:7) gave pure 1-(2,3-dihydroxy-1-methoxypropyl)-5-fluorouracil (0.62 g, 53%) with m.p. 155.5—161°C; $\nu_{max}$(KBr) 1710, 1664, 1250, 1080 $cm^{-1}$; $^1$Hn.m.r. [$(CD_3)_2SO$] $\delta$3.45 (3H, d,$CH_3O$), 3.50—4.10 (3H, m, $CH_2$ and CH), 4.50—5.30 (2H, br.s, 2x OH. $D_2O$-exchangeable), 5.50—5.75 (1H, m, CH), 7.80 (1H, d, $C_6H$), 10.90 (1H, br.s, NH, $D_2O$-exchangeable); m/e (70 eV) 234 ($M^+$, 10%). *Anal.* Calcd. for $C_8H_{11}N_2O_5F$: C, 41.02; H, 4.70; N, 11.96. Found: C, 40.99; H, 4.95; N, 11.90%

Example 3

*1-(3-Acetoxy-1-methoxypropyl)-5-fluorouracil*

5-Fluorouracil (6.6 g, 50.8 mmol) was treated with excess hexamethyldisilazane (100 mL) and a catalytic amount of solid ammonium sulphate at 160°C for 20 h. Removal of the excess silylating agent under reduced pressure afforded the bis-O-trimethylsilylated derivative as a pale brown mobile oil which was used immediately in the subsequent condensation.

The bis-trimethyl derivative was dissolved in anhydrous acetonitrile (250 mL) and treated under nitrogen with 1,3-diacetoxy-1-methoxypropane (10.6, 55.8 mmol). The resulting solution was cooled to −78°C and anhydrous stannic chloride (2 mL, 16.8 mmol) added. The mixture was stirred at −78°C for 15 minutes then slowly allowed to attain room temperature before stirring at 25°C for 4 h. The reaction mixture was reduced in volume to approximately 50 mL before cautious treatment with dilute sodium bicarbonate solution (250 mL.) The aqueous mixture was then extracted with chloroform (3 x 200 mL), organic phase dried ($MgSO_4$) and evaporated under reduced pressure, affording the crude reaction product as a pale cream solid. Recrystallization from acetone-hexane gave 1-(3-acetoxy-1-methoxypropyl)-5-fluorouracil (6.2 g 47%) as a white crystalline solid m.p. 123—125°C; $\nu_{max}$ (nujol) 3170, 1740, 1720, 1695, 1660, 1240 $cm^{-1}$; $^1$Hn.m.r. ($CDCl_3$) $\delta$2.08 (3H, s, ($CH_3CO$), 2.1 (2H, m, $CH_2$), 3.4 (3H, s, $CH_3O$), 4.18 (2H, t, J=6Hz, $CH_2OCO$), 5.76 (1H, t, J=6Hz, CH), 7.4 (1H, d, J=6Hz, $H^6$), 10.07 (1H, br.s, $D_2O$ exchangeable, NH).

*Anal.* Calcd. for $C_{10}H_{13}F\ N_2O_5$: C, 46.15, H, 5.04 N, 10.77
Found: C, 45.93, H, 5.10, N, 10.71%

Example 4

*1-(3-Hydroxy-1-methoxypropyl)-5-fluorouracil*

1-(3-Acetoxy-1-methoxypropyl)-5-fluorouracil (5 g, 19.2 mmol) was dissolved in methanol (60 mL) and treated with 0.88 ammonia solution (180 mL) before being stirred at 25°C for 20 h. The methanol and water were removed under reduced pressure affording the crude reaction product as a yellow oil. Direct recrystallization from hot chloroform-cyclohexane gave 1-(3-hydroxy-1-methoxypropyl)-5-fluorouracil (3.7 g, 88%) as a white crystalline solid m.p. 131—133°C; $\nu_{max}$ (nujol) 3430, 3150, 1710, 1680, $cm^{-1}$; $^1H$ n.m.r. $[(CD_3)_2SO]$ $\delta$1.9 (2H, m, $CH_2$), 3.21 (3H, s, $CH_3O$), 3.45 (2H, m, collapses to t on $D_2O$ exchange, J=6Hz, $CH_2OH$), 4.48 (1H, m, $D_2O$ exchangeable, OH), 5.63 (1H, t, J=6Hz, CH) 7.9 (1H, d, J=7Hz, $H^6$), 11.76 (1H, br.s, $D_2O$ exchangeable), NH); m/e 218 ($M^+$, 2%).

*Anal.* Calcd. for $C_8H_{11}FN_2O_4$: C, 44.04, H, 5.08, N, 12.84.
Found: C, 44.02, H, 5.17, N, 12.67%.

Preparation of Intermediate Compounds

Description 1

(a) *1,3-Dichloro-1-methoxypropane*

Hydrogen chloride gas was passed through a mixture of acrolein (11.5 g, 0.2 mmol) and methanol (6.6 g, 0.2 mmol) at 0°C until 16 g (0.44 mmol) had been taken up. The mixture was held at 0°C for a further 20 minutes before the larger bottom layer was separated. This organic layer was dried over calcium chloride and distilled under reduced pressure to give 1,3-dichloro-1-methoxypropane (17.0 g, 58%) as a clear liquid, b.p.$_{20}$ 46—52°C; $^1$Hnmr ($CDCl_3$) $\delta$2.43 (2H,q,J=4Hz, $CH_2CH$), 3,53 (3H,s,$CH_3$), 3.66 (2H,t,J=4Hz, $CH_2Cl$), 5.65 (1H,t,J=4Hz,CH).

(b) *1,3-Diacetoxy-1-methoxypropane*

Potassium acetate (18.6 g, 0.19 mmol) was added to a solution of 1,3-dichloro-1-methoxypropane (13.6, 0.096 mmol) in dry acetonitrile (400 mL) and stirred for 2 hours (Solution A). Potassium acetate (9.3 g, 0.095 mmol) was added to a solution of 18-crown-6 (25 g, 0.095 mmol) in acetonitrile (500 mL) and stirred for 2 hours (Solution B). Solutions A and B were mixed and the resulting solution refluxed for 18 hours before filtration and removal of the acetonitrile under reduced pressure. The residue was dissolved in ether (1 L) washed with water 3 × 500 mL) and dried ($MgSO_4$). Removal of the ether under reduced pressure afforded a yellow liquid which was distilled under reduced pressure to give 1,3-diacetoxy-1-methoxypropane (6.8 g, 45%) as a clear liquid, b.p$_{30}$ 130—5°C; $\nu_{max}$(film) 1760 $cm^{-1}$; 1Hnmr ($CDCl_3$) $\delta$2.0 (2H,m,$CH_2$), 2.05 (3H,s,$COCH_3$), 2.1 (3H,s,$COCH_3$), 3.4 (3H,s,$OCH_3$), 4.2 (2H,t,J=8Hz,$CH_2O$), 5.8 (1H,t,J=6Hz,CH).

Description 2

(a) *Glyceraldehyde Dimethyl Acetal*

A mixture of acrolein dimethyl acetal (306 g, 3mmol) in water (3.6 L) was cooled to 5°C, and a solution of analar potassium permanganate (480 g, 3 mmol) in water (9.0 L) added at a rate of approximating to 25 mL/min, ensuring that the reaction temperature was maintained between 3—5°C throughout the time of addition. After completion addition of the potassium permanganate solution, the mixture was allowed to stand at ≈5°C for 2 hours (the mixture became gel-like in consistency), and then heated on a steam bath for 1 hour.

The reaction mixture was then filtered and the manganese dioxide residue washed with water (1.5 L). The filtrate (≈13.5 L) was cooled to 0°C and anhydrous potassium carbonate (7.20 Kg) added, ensuring the temperature did not rise above 5°C. The two layers formed were separated and the water layer continuously extracted with ethyl acetate for 48 hours. The organic solutions were combined and dried ($MgSO_4$). The ethyl acetate was removed under reduced pressure (bath temp 35—40°C) yielding glyceraldehyde dimethyl acetal (183.6 g; 45% yield): $\nu_{max}$(film) 3420, 2940, 2835, 1070 $cm^{-1}$; $^1$Hnmr [$CDCl_3$] $\delta$3.45 (6H,d,2 × $CH_3O$), 3.50—3.90 (3H, br.s, $CH_2OH$ and $CHOH$), 4.10—4.40 (3H,m,$CH(OCH_3)_2$ and 2 × OH, $D_2O$-exchangeable); m/e (ammonia C.I.) 154 ($MNH_4^+$,38%),137 ($MH^+$, 19 105 ($M^+$—$OCH_3$, 50).

(b) *2,3-Diacetoxy-1,1-dimethoxypropane*

To a solution glyceraldehyde dimethyl acetal (117 g, 0.8 6 mmol) in dry pyridine (2 L) at 0°C (icebath) was added dropwise acetic anhydride (500 g, 5.50 mL). After complete addition the reaction was allowed to attain 25°C and stirred for 2 hours.

The mixture was then poured on to ice water (15 L) and the aqueous mixture extracted with methylene chloride (2 × 5 L). The organic phase was washed successively with dilute hydrochloric acid (10 L), dilute sodium hydrogen carbonate solution (2 × 10 L) and water (10 L) and then dried ($MgSO_4$). Removal of the solvent under reduced pressure gave the title product as a chromatographically (t.l.c.) homogeneous oil (170 g, 90% yield) with $\nu_{max}$(film) 1745, 1240, 1220, 1070 $cm^{-1}$; $^1$Hnmr ($CDCl_3$) $\delta$2.05 (2H,s,$CH_3CO_2$), 2.10 (3H,s,$CH_3CO_2$), 3.35—3.40 (6H,d,2 × $CH_3O$), 3.95—4.50 (3H,m,$CH_2$ and $CH(OCH_3)_2$), 5.05—5.25 (1H,m,CH).

(c) 1,2,3-Triacetoxy-1-methoxypropane

2,3-Diacetoxy-1,1-dimethoxy-propane (11 g. 50 mmol) and acetic anhydride (13.3 g, 130 mmol) were stirred together in the presence of concentrated sulphuric acid (1 drop) at room temperature for

24 hours. The mixture was poured onto ice (100 mL) and extracted with chloroform (3 x 80 mL). The chloroform layer was washed with dilute sodium bicarbonate solution (2 x 100 mL), water (100 mL) and dried ($CaCl_2$). The organic layer was evaporated under reduced pressure affording a pale lemon liquid, which was chromatographed on silica eluting with chloroform. 1,2,3-Triacetoxy-1-methoxypropane (25.9 g, 92%) was isolated as a clear liquid. $\nu_{max}$(film) 1740, 1440, 1370, 1220 $cm^{-1}$; $^1$Hnmr ($CDCl_3$) $\delta$2.1 (9H,s, 3 x $CH_3CO$), 3.47 (3H, s, $CH_3O$), 4.3 (2H,m,$CH_2$), 5.23

$$(1H,m,\mathit{CH}O\underset{\underset{O}{\|}}{C}CH_3),$$

5.88 (1H,m,$\mathit{CH}OCH_3$); m/e 266 ($MNH_4^+$, 28%), 189 (100).

Demonstration of Effectiveness of Compounds

Testing Procedure

Groups of 5 female Balb/c mice were inoculated intraperitoneally with $10^6$ Ehrlich ascites tumour cells. The compounds of Example 2 and 4 were each administered intraperitoneally at a range of single daily doses from 25 mg/kg—800 mg/kg from the third to the seventh day after inoculation and survival times recorded.

The results are given in the following Tables.

TABLE 1 Example No. 2.

| Dose | Number of Animals | Days of Treatment | Days of Death | Range of Days of Death | Average | % Increase in Survival Time Over Control |
|---|---|---|---|---|---|---|
| – | 10 | – | 18, 18, 18, 19, 19, 19, 20, 20, 20 | 18–20 | 19.0 | 0 % |
| 50 mg/kg | 5 | 3–7 | 20, 20, 24, 28, 31 | 20–31 | 24.6 | 29.5 % |
| 100 mg/kg | 5 | 3–7 | 18, 20, 20, 23, 33 | 18–33 | 22.8 | 20.0 % |
| 200 mg/kg | 5 | 3–7 | 19, 19, 20, 21, 21 | 19–24 | 20.6 | 8.4 % |
| 400 mg/kg | 5 | 3–7 | 18, 18, 33, 35, 36 | 18–36 | 28.0 | 47.4 % |
| 800 mg/kg | 5 | 3–7 | 19, 21, 21, 33, 35 | 19–35 | 25.8 | 35.8 % |

TABLE 2 – Example No. 4.

| Dose | Number of Animals | Days of Treatment | Days of Death | Range of Days of Death | Average | % Increase in Survival Time Over Control |
|---|---|---|---|---|---|---|
| – | 10 | – | 18, 18, 18, 19, 19, 19, 19, 20, 20, 20 | 18–20 | 19.0 | 0 % |
| 50 mg/kg | 5 | 3–7 | 19, 21, 21, 24, 24 | 19–24 | 21.8 | 14.7 % |
| 100 mg/kg | 5 | 3–7 | 20, 21, 21, 24, 24 | 20–24 | 22.0 | 15.8 % |
| 200 mg/kg | 5 | 3–7 | 24, 24, 31, 33, 35 | 24–35 | 29.4 | 54.7 % |
| 400 mg/kg | 5 | 3–7 | 19, 19, 20, 21, 21 | 19–21 | 20.0 | 5.3 % |
| 800 mg/kg | 5 | 3–7 | 19, 20, 20, 20, 31 | 19–31 | 22.0 | 15.8 % |

Summary of Results

The compounds of Examples 2 and 4 each required 400 mg/kg and 200 mg/kg doses, respectively, for 50% increase in survival time compared to untreated animals. Neither compound was appreciably toxic at doses up to 800 mg/kg and highest level tested.

## Claims

1. A compound of formula (I):

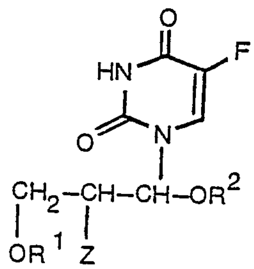

(I)

wherein

$R^1$ is hydrogen or $C_{1-6}$ alkanoyl

$R^2$ is $C_{1-6}$ alkyl and

Z is hydrogen or a group $-OR^1$

2. A compound as claimed in claim 1 wherein $R^2$ is methyl and $R^1$ is acetyl or hydrogen.

3. A compound as claimed in claim 1 or claim 2 wherein Z is hydrogen or hydroxy and $R^1$ is hydrogen.

4. 1-(2,3-Dihydroxy-1-methoxypropyl)-5-fluorouracil;

5. 1-(3-hydroxy-1-methoxypropyl)-5-fluorouracil.

6. A process for producing a compound of formula (I) as defined in claim 1 which process comprises reacting a compound of formula (II):

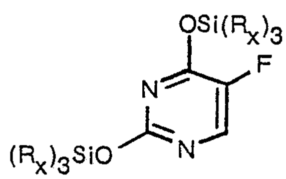

(II)

wherein $R_x$ is $C_{1-4}$ alkyl

with a compound of formula (III):

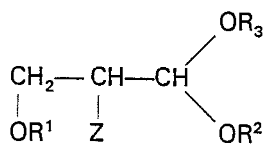

(III)

wherein

$R^1$ is $C_{1-6}$ alkanoyl

$R^2$ is $C_{1-6}$ alkyl

Z is hydrogen or a group $OR^1$ and

$R^3$ is $C_{1-6}$ alkyl or $C_{1-6}$ alkanoyl

in the presence of a Lewic acid.

7. A process as claimed in claim 6 wherein the Lewis acid is tin tetrachloride and the reaction is conducted in anhydrous acetonitrile under an atmosphere of dry nitrogen.

8. A pharmaceutical composition comprising a compound of formula (I) as defined in claim 1 in association with a pharmaceutically acceptable carrier or excipient.

9. A composition as claimed in claim 8 in unit dosage form comprising from 50 mg to 1 g of the compound of formula (I) per unit dose.

10. A compound of formula (I) as defined in claim 1 for use in treating the human or animal body.

## Patentansprüche

1. Eine Verbindung der Formel (I):

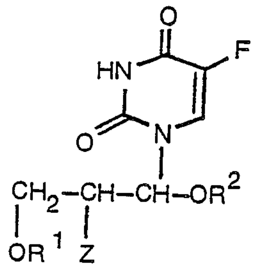

(I)

in welcher
$R^1$ Wasserstoff oder ein $C_{1-6}$-Alkanoyl,
$R^2$ ein $C_{1-6}$-Alkyl, und
Z Wasserstoff oder eine Gruppe $—OR^1$
bedeuten.

2. Eine Verbindung wie in Anspruch 1 beansprucht, in welcher $R^2$ Methyl bedeutet und $R^1$ Acetyl oder Wasserstoff ist.

3. Eine Verbindung wie in Anspruch 1 oder 2 beansprucht, in welcher Z Wasserstoff oder Hydroxy ist und $R^1$ Wasserstoff bedeutet.

4. 1-(2,3-Dihydroxy-1-methoxypropyl)-5-fluorouracil.

5. 1-(3-Hydroxy-1-methoxypropyl)-5-fluorouracil.

6. Ein Verfahren zur Herstellung einer Verbindung der Formel (I) wie in Anspruch 1 definiert, welches Verfahren die Umsetzung einer Verbindung der Formel (II):

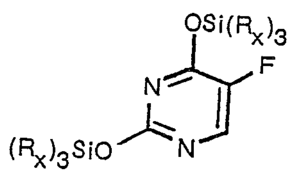

(II)

in welcher $R_x$ ein $C_{1-4}$-Alkyl ist,
mit einer Verbindung der Formel (III):

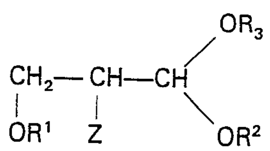

(III)

in welcher
$R^1$ ein $C_{1-6}$-Alkanoyl,
$R^2$ ein $C_{1-6}$-Alkyl,
Z Wasserstoff oder eine Gruppe $—OR^1$ und
$R^3$ ein $C_{1-6}$-Alkyl oder $C_{1-6}$-Alkanoyl ist,
in Gegenwart einer Lewissäure, umfaßt.

7. Ein Verfahren wie in Anspruch 6 beansprucht, in welchem die Lewissäure Zinntetrachlorid ist, und die Reaktion in wasserfreiem Acetonitril in einer Atmosphäre von trockenem Stickstoff durchgeführt wird.

8. Eine pharmazeutische Zusammensetzung umfassend eine Verbindung der Formel (I) wie in Anspruch 1 beansprucht, in Vereinigung mit einem pharmazeutisch verträglichen Träger bzw. Arzneimittelträger.

9. Eine Zusammensetzung wie in Anspruch 8 beansprucht, in Einzeldosisform umfassend 50 mg bis 1g der Verbindung der Formel (I) pro Einzeldosis.

10. Eine Verbindung der Formel (I) wie in Anspruch 1 definiert zur Verwendung bei der Behandlung des menschlichen oder tierischen Körpers.

**Revendications**

1. Composé de formule (I):

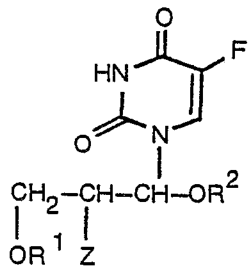

(I)

dans laquelle
$R^1$ est de l'hydrogène ou un alcanoyle en $C_{1-6}$;
$R^2$ est un alcoyle en $C_{1-6}$ et
Z est de l'hydrogène ou un groupe —$OR^1$.

2. Composé suivant la revendication 1, caractérisé en ce que $R^2$ est un méthyle et $R^1$ est un acétyle ou de l'hydrogène.

3. Composé suivant la revendication 1 ou 2, caractérisé en ce que Z est de l'hydrogène ou un hydroxy et $R^1$ est de l'hydrogène.

4. 1-(2,3-Dihydroxy-1-méthoxypropyl)-5-fluoro-uracile.

5. 1-(3-Hydroxy-1-méthoxypropyl)-5-fluoro-uracile.

6. Procédé pour la production d'un composé de formule (I) tel que défini dans la revendication 1, ce procédé consistant à faire réagir un composé de formule (II):

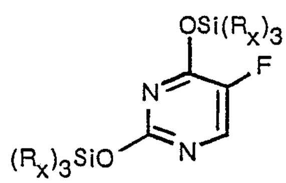

(II)

dans laquelle $R_x$ est un alcoyle en $C_{1-4}$, avec un composé de formule (III):

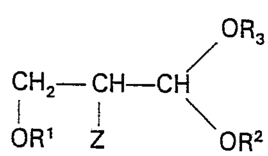

(III)

dans laquelle
$R^1$ est un alcanoyle en $C_{1-6}$;
$R^2$ est un alcoyle en $C_{1-6}$;
Z est de l'hydrogène ou un groupe $OR^1$; et
$R^3$ est un alcoyle en $C_{1-6}$ ou un alcanoyle en $C_{1-6}$,
en présence d'un acide de Lewis.

7. Procédé suivant la revendication 6, caractérisé en ce que l'acide de Lewis est du tétrachlorure d'étain et la réaction est effectué dans l'acétonitrile anhydre sous une atmosphère d'azote anhydre.

8. Composition pharmaceutique renferment un composé de formule (I) suivant la revendication 1, associé à un véhicule ou excipient pharmaceutique acceptable.

9. Composition suivant la revendication 8, sous forme posologique unitaire renferment de 50 mg à 1 g du composé de formule (I) par dose unitaire.

10. Composé de formule (I) suivant la revendication 1, destiné à être utilisé pour le traitement du corps humain ou des animaux.